# EUROPÄISCHE PATENTANMELDUNG

(11) **EP 1 935 973 A1**
(43) Veröffentlichungstag der Anmeldung: **25.06.2008**
(21) Anmeldenummer: 07076081.4
(22) Anmeldetag: 12.12.2007
(51) Int. Cl.: C12M 1/04, C12M 1/02

(54) **Kulturvorrichtung für aquatische Organismen**

(30) Priorität: 23.12.2006 DE 102006062634
(71) Anmelder: Stiftung Alfred-Wegener-Insitut für Polar und Meeresforschung, 27570 Bremerhaven (DE)
(72) Erfinder: Wiegemann, Maja, Dr., 27498 Helgoland (DE); Dunker, Erich, 27607 Langen (DE)

(57) **Zusammenfassung**

Solche Kulturvorrichtungen zur Vermehrung und Untersuchung von im Wasser lebenden Organismen bestehen aus einem verschließbaren Gefäß zu Besatz, Ernte, Versorgung und Temperierung von Kulturorganismen, und der Durchmischung von Wasser, Nährmedien und Gasen. Die erfindungsgemäße Kulturvorrichtung weist ein schlankes Gefäß (TG) aus transparentem Material mit einem Deckel (DE) mit einer Öffnung (OE) zum Befüllen mit Wasser, anderen Flüssigkeiten und Nährmedien und mit einem Abfluss (AB) mit Ventil (AV) im verjüngten Teil des Gefäßbodens (VB) auf. Ein Gasversorgungsanschluss (AZ) mit Rückschlagventil (RV) unten und eine Durchmischungseinrichtung sind integriert und zur Herstellung gerichteter Strömung oder Zirkulation und zum Betrieb im Durchflussverfahren geeignet. Das röhrenförmige Gefäß (GT) mit geringem Volumen lässt sich gut beheizen. Die Begasung am unteren Gefäßende (VB) erzeugt eine das Kulturmedium (KM) im Gefäß (TG) vollständig erfassende Strömung und eine gleichmäßige Versorgung mit Nährstoffen und Gasen. Ein Absinken von Nährmedien wird verhindert. Durch die vollständige Durchmischung des Kulturmediums (KM) sowie die sichere Bindung der Atemgase daran ist eine separate Mischeinrichtung oder ein Umpumpen überflüssig.

## Beschreibung

Die Erfindung bezieht sich auf eine Kulturvorrichtung für aquatische Organismen mit einem sich nach unten verjüngenden, transparenten, mit Wasser befüllbaren und verschließbaren Gefäß mit Einrichtungen
- zum Besatz mit Organismen oder besiedelten Substraten,
- zur Entnahme von Ernteorganismen,
- zur Versorgung mit Gasen und Nährmedien,
- zur Temperierung und
- zur Herstellung und Aufrechterhaltung einer Durchmischung von Wasser, Nährmedien und Gasen zumindest im Betrieb als geschlossenes System.

Derartige Kulturvorrichtungen werden zur Vermehrung und Untersuchung von im Wasser lebenden Klein- und Mikroorganismen verwendet. Das transparente Gefäß sorgt für die zum Leben notwendige und dosierbare Lichtmenge, es kann mit Süß- oder Salzwasser beliebiger Konzentration befüllt werden. Über die darüber hinaus steuerbare Zufuhr von Gasen und Nährmitteln können die Abläufe der diversen Lebensprozesse der Besatzorganismen, insbesondere ökologische Themen zur Nahrungslimitierung und Stressinduzierung bei aquatischen Organismen, z.B. Biofilmen, gesteuert und untersucht werden. Eine kommerzielle Anwendung für eine solche Kulturvorrichtung ist beispielsweise die Zucht von Artemia, Salzwasserkrebsen, die zur Verwendung als Zierfischfutter in allen Reifestadien von der Larve bis zum erwachsenen Krebs gerne gezüchtet werden.

### Stand der Technik

Aus der DE 196 48 875 B4 ist ein Fermenter für Biomasse bekannt, der einen Tank als Kulturgefäß mit Ein- und Auslassöffnungen für das Kulturmedium und Besatzorganismen sowie eine Rühreinrichtung zur ständigen Bewegung der Biomasse in seinem Inneren aufweist. Der Fermenter ist durch Wärmezufuhr über ein mit der Rühreinrichtung verbundenes Heiz- und Kühlelement temperierbar. Fermenter solcher Bauart sind für den kontinuierlichen Betrieb im industriellen Maßstab vorgesehene Bioreaktoren zur Erzeugung von Biomasse und Biogas. Die Mischung der Bestandteile des Kulturmediums wird durch Rühren mit elektrisch betriebenen Rühreinrichtungen bewirkt. Eine Separation erwünschter von unerwünschten Bestandteilen der Kulturorganismen ist nur in nachgeschalteten Einrichtungen außerhalb des Fermenters möglich.

Aus der DE 6812693 ist ein Artemia-Salina-Kulturgerät bekannt, das aus einem einfachen, zylinderförmigen und unten sich verjüngenden transparenten Gefäß besteht. Es wird mit Wasser und Nährmitteln befüllt und nach dem Besatz mit Artemia-Eiern mit einem Deckel verschlossen. Im Deckel befindet sich eine Öffnung, durch die ein bis fast auf den Boden des Gefäßes reichendes Rohr geführt ist. Durch das Rohr wird die zur Atmung der Organismen benötigte Luft geleitet. Die aufsteigenden Luftblasen sorgen nach dem Gegenstromprinzip für die Bindung der Luft an das Wasser und darüber hinaus für die Bewegung der Eier, die sich durch Schwerkraft immer wieder im sich verjüngenden unteren Bereich des Gefäßes sammeln. Durch eine weitere Öffnung im Deckel wird die oben austretende Luft an die Atmosphäre abgeführt. Zur weiteren Nährmittelzuführ im Wachstumszyklus muss der Deckel geöffnet werden. Zur Entnahme der fertig gereiften Organismen muss der Deckel entfernt und das Wasser abgegossen werden. Dabei können die Organismen abgefiltert, die Eischalen jedoch so nicht separiert werden. In einer anderen Ausführung des Kulturgeräts erfolgt die Luftzufuhr direkt durch den Gefäßboden.

Die DE 36 39 368 A1**,** von der die vorliegende Erfindung als nächstliegendem Stand der Technik ausgeht, offenbart ein Verfahren und eine Vorrichtung zur Kultivierung matrixgebundener, biologischer Zellsysteme. Das Kultivierungsverfahren wird in einer Vorrichtung durchgeführt, die aus einem in zwei Kammern unterteilten Vorratsgefäß und einem kugelförmigen Kulturgefäß besteht. Beide Gefäße sind durch einen Verteilerdeckel verbunden, der einerseits rohrförmige Öffnungen zwischen den Kammern des Vorratsgefäßes und dem Kulturgefäß und andererseits eine seitliche Öffnung zur Befüllung des Kulturgefäßes mit Besatzorganismen oder Nährmitteln aufweist. Das Vorratsgefäß ist seinerseits oben mit einem Deckel abgeschlossen, der je eine Öffnung in die beiden Kammern enthält. Zum Betrieb wird das Kulturgefäß und ca. zwei Drittel der Kammern des Vorratsgefäßes von oben mit dem Kulturmedium befüllt. Durch die seitliche Öffnung des Kulturgefäßes wird dieses mit den Besatzorganismen versehen. Durch abwechselndes Bepumpen mit Luft oder einem anderen Atemgas durch die Öffnungen im Deckel des Vorratsgefäßes wird Kulturmedium aus der gerade bepumpten Kammer über die zugehörige rohrförmige Öffnung in das Kulturgefäß gedrückt, sorgt dort für Bewegung und drückt weiterhin Kulturmedium durch die andere rohrförmige Öffnung in die andere Kammer und damit Atemgas durch die andere Öffnung im Deckel zurück und umgekehrt. Das bei diesem aufwändigen Pumpvorgang zugeführte Atemgas muss bei den folgenden Strömungsbewegungen des Kulturmediums in das Kulturgefäß an die Besatzorganismen gebracht werden. Für eine effektive Bindung des Atemgases an das Kulturmedium ist eine Oberflächenvergrößerung zwischen Atemgas und Kulturmedium erforderlich, die bei dieser Vorrichtung durch Glaskugeln oder Glasröhren in den Kammern des Vorratsgefäßes erzielt wird. Zur besseren Durchmischung im Kulturgefäß werden düsenartige Verlängerungen an den Öffnungen im Verteilerdeckel bis zum Boden des Kulturgefäßes vorgeschlagen. Sowohl das Verfahren mit ständigen Wechsel der Pumprichtung als auch die Vorrichtung mit ihren insgesamt drei Gefäßteilen ist aufwendig in Herstellung und Betrieb. Zum Hinzufügen von weiteren Nährmitteln für die Versorgung der Besatzorganismen über die Erstausstattung des Kulturmediums hinaus, muss der Betrieb unterbrochen und der Verschluss der seitlichen Öffnung im Verteilerdeckel geöffnet werden. Zur Ernte der kultivierten Organismen muss die Vorrichtung demontiert und das Kulturmedium im Kulturgefäß abgegossen und gefiltert werden. Dabei ist auch hier eine Separierung von Ernteorganismen und z.B. Eierschalen nicht unmittelbar gegeben.

### Aufgabenstellung

Die **Aufgabe** für die vorliegende Erfindung ist es daher, eine Kulturvorrichtung für aquatische Organismen zur Verfügung zu stellen, die eine einfache Versorgung mit Besatzorganismen, Kulturmedium, Nährmitteln und Atemgasen, eine einfache, möglichst unterbrechungsfreie Ernte der Kulturorganismen, ggf. mit Separierung unerwünschter Bestandteile, die Herstellung einer gerichteten Strömung, die Nutzung im Durchflussbetrieb oder als geschlossenes System, einen zumindest teilweise automatisierbaren Betrieb und eine einfache Handhabung ermöglicht. Die erfindungsgemäße Lösung für diese Aufgabe ist dem Anspruch zu entnehmen.

Die erfindungsgemäße Kulturvorrichtung für aquatische Organismen ist dadurch gekennzeichnet, dass
- das transparente Gefäß am oberen Ende einen Deckel mit einer verschließbaren Öffnung zum Befüllen und am unteren Ende einen Abfluss mit Ventil aufweist,
- die Einrichtung zur Versorgung mit Gasen ein Anschluss mit Rückschlagventil am unteren Ende des transparenten Gefäßes ist,
- die Einrichtung zur Herstellung und Aufrechterhaltung einer Durchmischung von Wasser, Nährmedien und Gasen mit der Einrichtung zur Versorgung mit Gasen identisch ist und
- die Einrichtung zur Herstellung und Aufrechterhaltung einer Durchmischung von Wasser, Nährmedien und Gasen auch Einrichtungsbestandteile zur gerichteten Strömung oder Zirkulation und zum Betrieb im Durchflussverfahren aufweist.

Das schlanke Gefäß aus transparentem Material wie Glas oder Plexiglas wird an seinem oberen Ende von einem Deckel verschlossen, in dem eine Öffnung mit einem Anschluss für die direkte Zufuhr von Wasser, anderen Flüssigkeiten und Nährmedien vorgesehen ist. Sein unteres Ende verjüngt sich bis zum Abfluss mit Ventil zum Ablassen der im Gefäß vorhandenen Flüssigkeiten bei Bedarf. Der röhrenartige Bau des Gefäßes bedingt eine gute Raumnutzung, wodurch das nötige Volumen des Mediums gering gehalten wird. Die Einbringung von Gas nahe des Auslasses des Gefäßes ermöglicht die Erzeugung einer Strömung, welche das Kulturmedium im Gefäß vollständig erfasst. Das sorgt für eine gleichmäßige Versorgung der besiedelten Substrate mit Nährstoffen, Gasen usw. Ein Absinken von Nährmedien wird verhindert. Aufgrund der effizienten Nutzung aller Stoffe wird das Wartungsintervall verlängert. Im Gegensatz zum Stand der Technik wird bei der vorliegenden Erfindung die vollständige Durchmischung der Bestandteile des Kulturmediums und der ggf. frei schwebenden Kulturorganismen sowie die sichere Bindung der Atemgase an das Kulturmedium durch die Einleitung der Atemgase nahe des Auslasses am Boden des schlanken Gefäßes erreicht. Dadurch ist eine separate Mischeinrichtung oder ein dauerndes Umpumpen des Kulturmediums überflüssig.

Eine vorteilhafte Weiterbildung der Kulturvorrichtung für aquatische Organismen nach der Erfindung ergibt sich, wenn die Einrichtung zum Besatz mit Startorganismen eine Halterung für mit Substraten versehene Objekträger ist. Testsubstrate, die mit diversen Organismen besiedelt sein können, werden auf Objektträgern mit einer Halterung in das Gefäß eingesetzt. Die Halterung kann nach Entfernung des Deckels der Röhre jederzeit herausgezogen werden, um Substrate zu wechseln oder zu pflegen. Weiterhin kann es vorteilhaft sein, wenn die Einrichtung zur Entnahme von Ernteorganismen eine Falle aus einem beidseitig verschließbaren transparenten Rohr mit einer die Rückwanderung der Organismen verhindernden Barriere im Inneren am oberen Ende des transparenten Gefäßes ist. Sichtbares Licht oder andere Strahlung, für die das Material des Gefäßes transparent ist, kann beliebig von der Seite über die gesamte Länge des Gefäßes zugeführt werden. Bewuchsorganismen produzieren in der Regel positiv phototaktische Larven. Das Abfangen von Larven wird durch eine im oberen Teil der Röhre angebrachte Falle als Einrichtung zur Entnahme von Ernteorganismen ermöglicht. Nach Abstellen der Strömung können die Larven aufgrund von chemo- oder phototaktischen Reizen in die Falle gelockt werden. Eine rückwärtige Bewegung der Larven in die Röhre wird durch eine Barriere im oberen Teil der Falle verhindert. Ferner kann die Einrichtung zur Versorgung mit Nährmedien eine verschließbare und zum Anschluss von Dosierautomaten vorbereitbare Öffnung im Deckel sein. Die Zudosierung von Stoffen kann so durch den Anschluss von Dosierautomaten ggf. automatisiert werden. Außerdem ist es vorteilhaft, wenn die Einrichtung zur Temperierung eine in eine Öffnung im Deckel einsetzbare Heizung ist. Die Temperierung kann z.B. durch die Einführung einer Tauchheizung gewährleistet werden.

Besonders vorteilhafte Weiterbildungen der Kulturvorrichtung für aquatische Organismen nach der Erfindung ergeben sich, wenn die Einrichtung zur Versorgung mit Gasen mehrere, um den Abfluss gleichmäßig verteilt angeordnete Öffnungen oder einen Ringspalt aufweist oder wenn die Einrichtung zur Versorgung mit Gasen eine einzelne, zentral in dem Abfluss angeordnete Düse aufweist. Insbesondere diese letztere Anordnung kann ein Absinken von Stoffen und deren Ablagerung im Abfluss wirksam verhindern. Schließlich kann es noch vorteilhaft sein, wenn zur Herstellung einer Zirkulation beim Betrieb als geschlossenes System das transparente Gefäß eine Trennscheibe aufweist. Die Trennscheibe wird so im transparenten Gefäß angeordnet, dass sich durch das Einströmen von Gasen eine eindeutig gerichtete Strömung aus einer Aufwärts- und einer Abwärtsbewegung der Nährflüssigkeit um die Trennscheibe herum ergibt. Zum Betrieb im Durchflussverfahren können die verschließbare Öffnung des Deckels am oberen Ende und der Abfluss mit Ventil am unteren Ende des transparenten Gefäßes eingesetzt werden. Eine außerhalb des transparenten Gefäßes angeordnete Pumpe sorgt dabei für die Umwälzung des angereicherten Nährmediums.

Die Wartung der Anlage kann durch eine automatische Überwachung von Parametern des Kulturmediums und die Kopplung an Dosiereinrichtungen vorgenommen werden. Ein kompletter Wechsel des Kulturmediums ist ebenfalls automatisch oder manuell möglich. Zum Ablassen des Mediums wird das Ventil am Auslass geöffnet, die Befüllung erfolgt nach Schließen des Ventils am Auslass. Spülzyklen können der Befüllung des Gefäßes vorgeschaltet werden.

Der Wechsel des Kulturmediums kann über eine Zeitschaltuhr geregelt werden. Der obere und der untere Teil des Gefäßes sind abschraubbar, so dass eine Grundreinigung erleichtert wird. Die Kulturvorrichtung kann auch im Durchflussmodus betrieben werden, wobei der Rückfluss über eine Pumpe erfolgen kann oder Kulturmedium aus einem Vorratsbecken in das Gefäß gepumpt wird. Entgegen der Flussrichtung aufsteigendes Gas erzeugt dabei einen Gegenstromeffekt. Durchfluss und Gegenstrom können den Bedürfnissen der Anwendung angepasst werden.

Zur Zucht von Bewuchsorganismen gilt generell, dass in der Zuchtröhre ideale Ernährungs- und Wachstumsbedingungen derart eingestellt werden können, dass das System für diverse Bewuchsorganismen genutzt werden kann, die ansonsten als filtrierende Organismen in Anlagen einen limitierten Zugriff auf Nahrung haben. Da ideale Bedingungen im Allgemeinen zügig zu Fortpflanzungsaktivitäten führen, ist in Kombination mit der Larvenfalle das Ernten von Larven möglich. Die Erzeugung von Seepockenlarven ist ein Spezialfall. Seepocken werden auf Objektträgern angesiedelt und nach Erreichen des fortpflanzungsfähigen Alters in die Röhre eingebracht. Die Seepocken werden hier unter geeigneten Bedingungen (Futter, Sauerstoff, Strömung, pH, Licht etc.) gehalten und zur Fortpflanzung und periodisch zur Abgabe der Nauplien (Larven) gebracht. Die Nauplien werden in der Larvenfalle gesammelt. Zum Studium von Bewuchsorganismen unter diversen Bedingungen werden auf Objektträgern gewachsene Biofilme oder Makroorganismen in das System eingebracht. Die Reaktion der Biofilmorganismen nach Einstellung bestimmter Bedingungen (z. B. pH, Licht, Nahrungslimitierung, Gassättigung, oder Umströmung mit Gas) kann studiert werden. Dabei kann bei Operation sowohl im Durchflusssystem, als auch im geschlossenen System mit Zirkulation um eine Trennscheibe, eine gerichtete Strömung simuliert werden.

### Ausführungsbeispiele

Ausbildungsformen der Kulturvorrichtung für aquatische Organismen nach der Erfindung werden nachfolgend zum weiteren Verständnis der Erfindung anhand der schematischen Figuren näher erläutert. Dabei zeigt
- **Figur 1**: die Kulturvorrichtung im Schnitt und
- **Figuren 2a,b**: alternative Ausführungen der Einrichtung zur Versorgung mit Gasen.

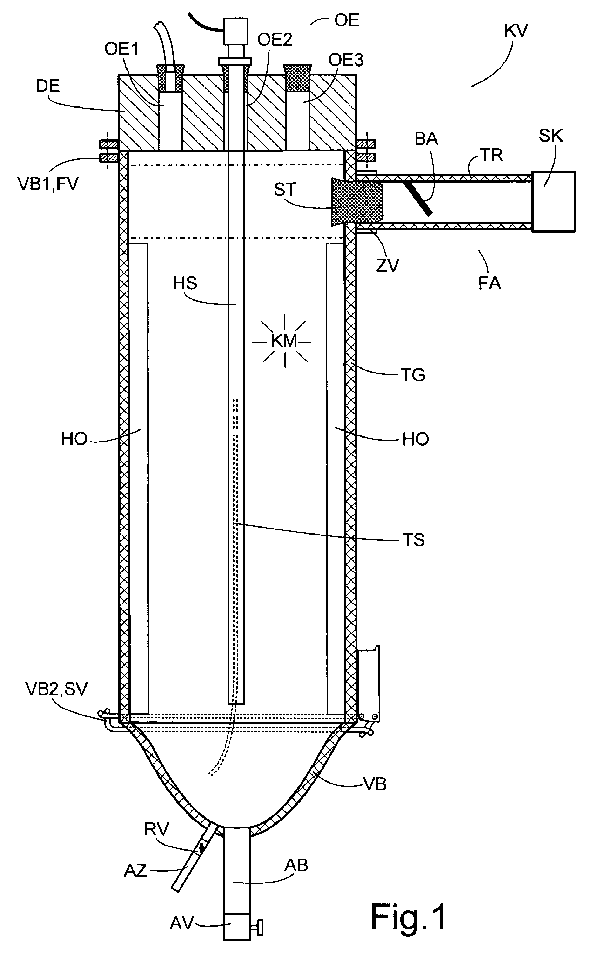

**Figur 1** zeigt den Aufbau einer Kulturvorrichtung für aquatische Organismen **KV** in einer Schnittdarstellung. Ein zentrales transparentes Gefäß **TG** wird oben durch einen Deckel **DE** abgeschlossen, der dazu eine erste Verbindung **VB1,** in der dargestellten Ausführungsform als einfache Flanschverschraubung **FV,** aufweist. Unten wird das transparente Gefäß **TG** durch einen sich verjüngenden Bodenabschnitt **VB** abgeschlossen, der dazu seinerseits eine weitere Verbindung **VB2,** in der dargestellten Ausführungsform als Schnellverschluss **SV,** aufweist. Beide Ausführungsformen stehen hier nur beispielhaft. Beide Verbindungen **VB1,VB2** sind gegen drucklose Flüssigkeiten abgedichtet. Im Inneren des transparenten Gefäßes **TG** ist die Halterung für Objektträger **HO** angeordnet. Die Objektträger können bei geöffnetem Deckel **DE** von oben in die Halterung für Objektträger **HO** eingeschoben und so fixiert werden. Der Deckel **DE** weist mehrere verschließbare Öffnungen **OE** zur Versorgung der Kulturvorrichtung für aquatische Organismen **KV** auf. Eine erste Öffnung **OE1** wird zur Befüllung mit dem Kulturmedium **KM** benötigt, durch eine zweite Öffnung **OE2** können Einrichtungen zur Regelung der Temperatur, z.B. Heizstäbe **HS,** oder anderer Parameter eingeführt werden. Eine dritte Öffnung **OE3** schließlich dient der Zugabe von Nährmitteln oder kann zum Anschluss von Dosiereinrichtungen für Nährmittel verwendet werden.

Zwischen dem Deckel **DE** und der Halterung für Objektträger **HO** ist die Falle **FA** für Larven der Kulturorganismen angeordnet. Sie ist als transparentes Rohr **TR** ausgeführt und direkt in das transparente Gefäß **TG** eingelassen, beispielsweise mit einer zentralen Verschraubung **ZV.** Das transparente Rohr **TR** ist am äußeren Ende mit einer Schraubkappe **SK** verschlossen. Am inneren Ende kann ein Stopfen **ST** eingesetzt werden, wenn der Deckel **DE** zur Ernte der Larven in der Falle **FA** entfernt wird. Die Falle **FA** weist im Inneren eine opake Barriere **BA** auf, die unter einem Winkel von etwa 45°nach vorne in das transparente Rohr **TR** hineinragt und unten nur einen Spalt offen lässt, der bei im Betrieb nicht eingesetztem Stopfen **ST** den Organismen den Eintritt in den Raum dahinter erlaubt, sie jedoch an der Rückkehr aus der Falle **FA** behindert. Wenn der Pegel des Kulturmediums **KM** bis unter den Anschluss der Falle **FA** aber oberhalb der Halterung für Objektträger **HO** durch Ablassen abgesenkt wird, bleibt der Objektträger **HO** mit dem Kultursubstrat weiter ungestört unter dem Kulturmedium **KM.** Am unteren Ende des sich verjüngenden Bodenabschnitts **VB** des transparenten Gefäßes **TG** ist ein Abfluss **AB** mit Absperrventil **AV** angeordnet. In seiner unmittelbaren Nähe ist ein Anschluss **AZ** für die Zufuhr von Atemgasen angeordnet, das ein Rückschlagventil **RV** zur Verhinderung des Eintritts von Kulturmedium **KM** in den Anschluss **AZ** aufweist. Eine Trennscheibe **TS** zur Herstellung einer gerichteten Zirkulation beim Betrieb im geschlossenen System ist gestrichelt angedeutet.

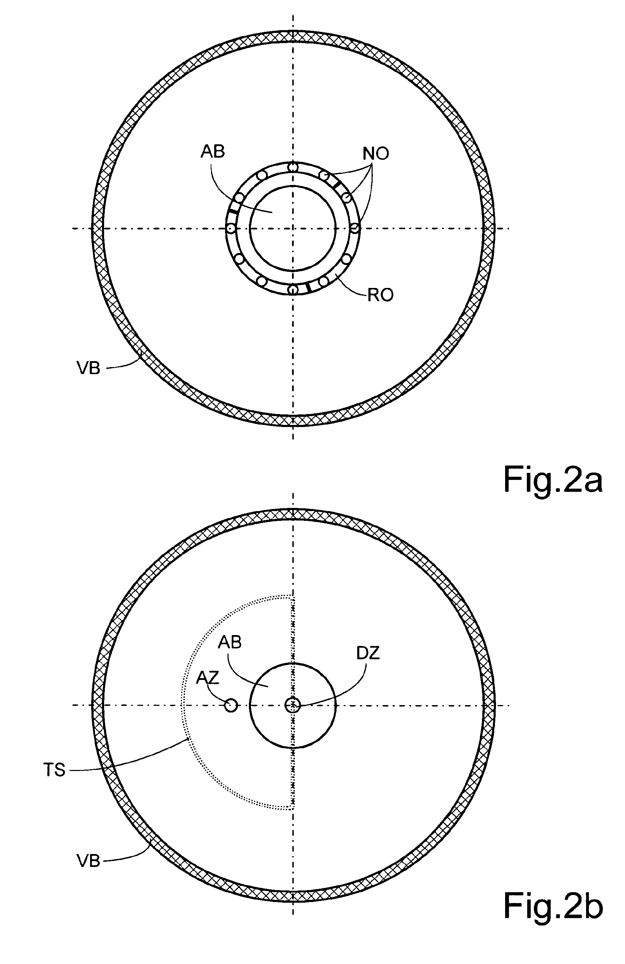

Die **Figuren 2a****,b** zeigen in der Draufsicht alternative Ausführungen der Einrichtung zur Versorgung mit Gasen, die zentral und symmetrisch zum Abfluss **AB** für eine gleichmäßig verteilte und das transparente Gefäß **TG** umfassend bestreichende Durchmischung des Kulturmediums **KM** gewährleisten.

Dabei zeigt **Figur 2a** zentral um den Abfluss **AB** herum gleichmäßig verteilt angeordnete Öffnungen **NO** bzw. zu einem geschlossenen Ringspalt **RO** vereinigte Öffnungen. **Figur 2b** zeigt alternativ dazu eine einzelne, zentral in dem Abfluss **AB** angeordnete Düse **DZ.** Alle alternativen Ausführungen der Einrichtung zur Versorgung mit Gasen, die immer identisch ist mit der Einrichtung zur Herstellung und Aufrechterhaltung einer Durchmischung von Wasser, Nährmedien und Gasen, bewirken eine besonders gleichmäßige und symmetrische Verteilung und Anlagerung der Gase im Kulturmedium **KM** sowie Durchmischung und Bewegung des Kulturmediums **KM** zusammen mit den frei beweglichen Organismen darin. Auch hier ist eine Trennscheibe **TS** zur Herstellung einer gerichteten Zirkulation beim Betrieb im geschlossenen System gestrichelt angedeutet.

### Bezugszeichenliste

- **AB**: Abfluss
- **AV**: Absperrventil
- **AZ**: Zufuhr von Atemgasen
- **BA**: Barriere
- **DE**: Deckel
- **DZ**: Düse
- **FA**: Falle
- **FV**: Flanschverschraubung
- **HO**: Halterung für Objektträger
- **HS**: Heizstab
- **KM**: Kulturmedium
- **KV**: Kulturvorrichtung für aquatische Organismen
- **NO**: Öffnung
- **OE**: verschließbare Öffnungen
- **RO**: Ringspalt
- **RV**: Rückschlagventil
- **SK**: Schraubkappe
- **ST**: Stopfen
- **SV**: Schnellverschluss
- **TG**: transparentes Gefäß
- **TR**: transparentes Rohr
- **TS**: Trennscheibe
- **VB**: sich verjüngender Bodenabschnitt
- **VB1**: erste Verbindung
- **VB2**: weitere Verbindung
- **ZV**: zentrale Verschraubung

## Patentansprüche

1. Kulturvorrichtung für aquatische Organismen mit einem sich nach unten verjüngenden, transparenten, mit Wasser befüllbaren und verschließbaren Gefäß mit Einrichtungen
- zum Besatz mit Organismen oder besiedelten Substraten,
- zur Versorgung mit Gasen und Nährmedien,
- zur Entnahme von Ernteorganismen,
- zur Temperierung und
- zur Herstellung und Aufrechterhaltung einer Durchmischung von Wasser, Nährmedien und Gasen zumindest im Betrieb als geschlossenes System,
**dadurch gekennzeichnet, dass**
- das transparente Gefäß (TG) am oberen Ende einen Deckel (DE) mit einer verschließbaren Öffnung (OE1) zum Befüllen und am unteren Ende einen Abfluss (AB) mit Ventil (AV) aufweist,
- die Einrichtung zur Versorgung mit Gasen ein Anschluss (AZ) mit Rückschlagventil (RV) am unteren Ende des transparenten Gefäßes (TG) ist,
- die Einrichtung zur Herstellung und Aufrechterhaltung einer Durchmischung von Wasser, Nährmedien und Gasen mit der Einrichtung zur Versorgung mit Gasen identisch ist und
- die Einrichtung zur Herstellung und Aufrechterhaltung einer Durchmischung von Wasser, Nährmedien und Gasen auch Einrichtungsbestandteile zur gerichteten Strömung oder Zirkulation und zum Betrieb im Durchflussverfahren aufweist.

2. Kulturvorrichtung nach Anspruch 1,
**dadurch gekennzeichnet, dass**
die Einrichtung zum Besatz mit Startorganismen eine Halterung (HO) für mit Substraten versehene Objekträger ist.

3. Kulturvorrichtung nach einem der Ansprüche 1 oder 2,
**dadurch gekennzeichnet, dass**
die Einrichtung zur Entnahme von Ernteorganismen eine Falle (FA) aus einem beidseitig verschließbaren transparenten Rohr (TR) mit einer die Rückwanderung der Organismen verhindernden Barriere (BA) im Inneren am oberen Ende des transparenten Gefäßes (TG) ist.

4. Kulturvorrichtung nach einem der Ansprüche 1 bis 3,
**dadurch gekennzeichnet, dass**
die Einrichtung zur Versorgung mit Nährmedien eine verschließbare und zum Anschluss von Dosierautomaten vorbereitbare Öffnung (OE3) im Deckel ist.

5. Kulturvorrichtung nach einem der Ansprüche 1 bis 4,
**dadurch gekennzeichnet, dass**
die Einrichtung zur Temperierung eine in eine Öffnung (OE2) im Deckel einsetzbare Heizung ist.

6. Kulturvorrichtung nach einem der Ansprüche 1 bis 5,
**dadurch gekennzeichnet, dass**
die Einrichtung zur Versorgung mit Gasen eine oder mehrere, im oder um den Abfluss (AB) verteilt angeordnete Öffnungen (NO) oder einen Ringspalt (RO) aufweist.

7. Kulturvorrichtung nach einem der Ansprüche 1 bis 6,
**dadurch gekennzeichnet, dass**
die Einrichtung zur Versorgung mit Gasen eine einzelne, zentral in dem Abfluss (AB) angeordnete Düse (DZ) aufweist.

8. Kulturvorrichtung nach einem der Ansprüche 1 bis 7,
**dadurch gekennzeichnet, dass**
das transparente Gefäß (TG) eine Trennscheibe (TS) zur Herstellung einer Zirkulation beim Betrieb als geschlossenes System aufweisen kann.

9. Kulturvorrichtung nach einem der Ansprüche 1 bis 8,
**dadurch gekennzeichnet, dass**
die Einrichtungsbestandteile zum Betrieb im Durchflussverfahren die verschließbare Öffnung (OE1) des Deckels (DE) am oberen Ende und der Abfluss (AB) mit Ventil (AV) am unteren Ende des transparenten Gefäßes (TG) sind.
